**Europäisches Patentamt**

**(19) European Patent Office**

**Office européen des brevets**

(11) Publication number : **0 326 103 B1**

(12)

# EUROPEAN PATENT SPECIFICATION

(45) Date of publication of patent specification :
**15.07.92 Bulletin 92/29**

(51) Int. Cl.⁵ : **A61K 31/415, A61K 9/48**

(21) Application number : **89101268.4**

(22) Date of filing : **25.01.89**

(54) **Pharmaceutical composition for 4-aroylimidazol-2-ones.**

(30) Priority : **29.01.88 US 149803**

(43) Date of publication of application :
**02.08.89 Bulletin 89/31**

(45) Publication of the grant of the patent :
**15.07.92 Bulletin 92/29**

(84) Designated Contracting States :
**AT BE CH DE ES FR GB GR IT LI LU NL SE**

(56) References cited :
**EP-A- 0 031 603**
**EP-A- 0 117 888**
**EP-A- 0 179 583**
**US-A- 4 405 635**

(73) Proprietor : **MERRELL DOW PHARMACEUTICALS INC.**
**2110 East Galbraith Road**
**Cincinnati Ohio 45215-6300 (US)**

(72) Inventor : **Domet, Jack**
**2158 Flowerwood Court**
**Cincinnati Ohio 45230 (US)**
Inventor : **Danielson, Douglas W.**
**424 Clay Road, Apt. B.**
**Rochester New York 14623 (US)**

(74) Representative : **Sgarbi, Renato et al**
**GRUPPO LEPETIT S.p.A. Patent and Trademark Department Via Roberto Lepetit, 34**
**I-21040 Gerenzano (Varese) (IT)**

## Description

Various 4-aroylimidazol-2-ones are disclosed in U.S. Patent 4,405,635 as compounds useful as antihypertensives, cardiotonics, antithrombotics, bronchodilators, and uterospasmolytics. Included within the scope of these generically defined 4-aroylimidazol-2-one compounds is 1,3-dihydro-4-methyl-5-[4-(methylthio)-benzoyl]-2H-imidazol-2-one which is an effective, orally-active cardiotonic agent. Since a typical 4-aroylimidazol-2-one compound is relatively water-insoluble, it is desirable to provide pharmaceutical compositions for these compounds which possess effective bioavailability characteristics.

US-A-4.405.635 reports examples of oral solid dosage and aqueous parenteral administration forms of 4-aroylimidazol-2-ones. EP-A-31603 discloses compositions containing nonionic surfactants for absorption of drugs, giving specific examples of formulations of 4-hexadecylaminobenzoic acid sodium salt as active ingredient useful for the treatment of atherosclerosis and hyperlipidemia.

Accordingly, the present invention provides a liquid pharmaceutical composition comprising a) an effective amount of a 4-aroylimidazol-2-one, or a pharmaceutically acceptable salt thereof, and b) one or more pharmaceutically acceptable nonionic surfactants in an amount from 30% to 99% by weight of the composition. Optionally, the composition can further include one or more pharmaceutically acceptable excipients other than nonionic surfactants comprising up to 45% by weight of the composition. The pharmaceutical compositions of the present invention possess effective bioavailability characteristics and are particularly useful wherein the 4-aroylimidazol-2-one employed is 1,3-dihydro-4-methyl-5-[4-(methylthio)-benzoyl]-2H-imidazol-2-one. According to the present invention, the term "liquid pharmaceutical composition" means and includes those compositions which are liquid such as a solution suspension or emulsion or the like at or below about 40 degrees centigrade (°C).

Various 4-aroylimidazol-2-ones, including 1,3-dihydro-4-methyl-5-[4-(methylthio)-benzoyl]-2H-imidazol-2-one, are disclosed in U.S. Patent 4,405,635, the complete disclosure of which is incorporated herein by reference.

According to the present invention, the term "nonionic surfactant" means and includes pharmaceutically acceptable nonionic surfactants which are known in the art of pharmaceutical science, including various nonionic compounds containing relatively hydrophilic and relatively hydrophobic regions. Typically these surfactants are alkoxylates of hydrophobic amines, acids or alcohols. For example, the term "pharmaceutically acceptable nonionic surfactants" is contemplated to include the following agents within its scope: various long chain fatty acid esters of polyoxyethylene sorbitan, such as Polyoxyethylene (20) Sorbitan monooleate, i.e. Polysorbate® 80 (also known as Tween® 80); polyethylene glycols of various average molecular weights, and derivatives thereof such as polyoxyethylene fatty acid esters; or mixtures thereof. Surfactants which are liquid at or below 40°C are generally preferred. The preferred nonionic surfactants in the pharmaceutical composition of the present invention are polyoxyethylene sorbitan fatty acid esters. Polysorbate® 80 is especially preferred.

The amount of nonionic surfactant in the pharmaceutical composition of the present invention can vary from 30% to 99% by weight of the composition. Compositions wherein the nonionic surfactant comprises from 55% to 75% by weight are preferred and those wherein the nonionic surfactant comprises from 60% to 70% by weight are especially preferred.

The pharmaceutical composition of the present invention can, in addition, optionally contain one or more pharmaceutically acceptable excipients other than nonionic surfactants. These excipients are therapeutically inert ingredients such as are well known and appreciated in the art. Such excipients include, for example, water-immiscible, volatile and non-volatile liquids (including vegetable oils such as soybean oil and aromatic oils and the like); water-miscible, non volatile liquids; water-miscible, relatively non-volatile compounds. Selection of a particular ingredient or ingredients and the amounts used can be readily determined by one of ordinary skill in the art by reference to standard procedures and practices.

Pharmaceutical compositions wherein pharmaceutically acceptable excipients other than nonionic surfactants are excluded, are generally preferred.

The liquid pharmaceutical composition of the present invention can be administered orally, in any suitable dosage form. The preferred dosage form for oral administration is a unit dosage form of a suitable capsule in which a pharmaceutical composition of the present invention has been encapsulated. Suitable capsules are those which are pharmaceutically acceptable and include both soft and hard gelatin capsules.

A liquid pharmaceutical composition of the present invention can generally be made by mixing the 4-aroylimidazol-2-one, or pharmaceutically acceptable salt thereof, in powder form with an amount of nonionic surfactant sufficient to thoroughly wet the powder. The remainder of nonionic surfactant, and any optional pharmaceutically acceptable excipients, can be added to the mixture and mixed until a uniform suspension is formed. Alternatively, the overall amounts of the ingredients can simply be mixed together using a suitable mixer. The suspension can optionally be deagglomerated by passing the material through a mill or other appropriate equipment adjusted to provide a smoother suspension.

The liquid pharmaceutical composition of the present invention can further be provided in solid unit dosage form by encapsulating an appropriate amount in pharmaceutically acceptable capsules such as either soft or hard gelatin capsules. The encapsulation can be accomplished according to procedures and conditions well known and appreciated in the art. It should be noted that the pharmaceutical compositions of the present invention can be relatively viscous in nature and that appropriate adjustments to parameters, as are well known and appreciated in the art, should be made in the encapsulation procedures so as to optimize the encapsulation process.

In a preferred embodiment, a pharmaceutical composition of the present invention can be prepared using a procedure analogous to that described in Example 1 to provide a solid unit dosage form for oral administration. This example is illustrative for the claimed composition and process.

EXAMPLE 1

Preparation of Capsules Containing 1,3-Dihydro-4-methyl-5-[4-(methylthio)-benzoyl]-2H-imidazol-2-one Suitable for Oral Administration

Combine 93 kilograms (kg) of 1,3-dihydro-4-methyl-5-[4-(methylthio)-benzoyl]-2H-imidazol-2-one (Compound A) and 100 kg of Polysorbate® 80 and mix in a suitable mixer until the powder is thoroughly wetted. To this mixture add an additional 82 kg of Polysorbate® 80 and mix until a uniform suspension is formed. Deagglomerate the suspension by passing through a Triple Roller Mill or other appropriate equipment for deagglomeration, adjusted to produce a smooth suspension suitable for encapsulation. Mix the suspension in a suitable mixer until the suspension is uniform. The composition of the suspension is about 33.8% by weight of Compound A and about 66.2% by weight of Polysorbate® 80.

Encapsulate the suspension into suitable soft gelatin capsule shells. Encapsulation of 37 milligrams (mg), 74 mg, 111 mg, 148 mg, 222 mg, 296 mg, and 444 mg of the suspension provides capsules containing 12.5 mg, 25 mg, 37.5 mg, 50 mg, 75 mg, 100 mg, and 150 mg, respectively, of Compound A.

**Claims**

**Claims for the following Contracting States : AT, BE, CH, DE, FR, GB, IT, LI, LU, NL, SE, S et A.**

1. A liquid pharmaceutical composition for use in oral administration dosage forms consisting of a) an effective amount of a 4-aroylimidazol-2-one, or a pharmaceutically acceptable salt thereof, and b) one or more pharmaceutically acceptable nonionic surfactants in an amount from 30% to 99% by weight of the composition.

2. A composition of Claim 1 wherein the 4-aroylimidazol-2-one is 1,3-dihydro-4-methyl-5-[4-(methylthio)-benzoyl]-2H-imidazol-2-one.

3. A composition of Claim 1, or 2 wherein a nonionic surfactant is a polyoxyethylene sorbitan fatty acid ester.

4. A composition of Claim 1, or 2 wherein a nonionic surfactant is Polyoxyethylene (20) sorbitan monoleate.

5. A composition of Claim 4 wherein the amount of the nonionic surfactant is from 55% to 75% by weight of the composition.

6. A composition of Claim 4 wherein the amount of the nonionic surfactant is from 60% to 70% by weight of the composition.

7. A composition of Claim 4 wherein the amount of the nonionic surfactant is 66% by weight of the composition.

8. A composition of one of Claim 5, 6 or 7 in unit dosage form of a pharmaceutically acceptable capsule.

9. A composition of any one of Claims 1 to 8 in a unit dosage form of a soft gelatin capsule.

10. A composition of any one of Claims 1 to 8 in a unit dosage form of a hard gelatin capsule.

11. A composition of any one of Claims 1 to 8 wherein the capsule contains 12.5 mg of 1,3-dihydro-4-methyl-5-[4-(methylthio)-benzoyl]-2H-imidazol-2-one.

12. A composition of any one of Claims 1 to 8 wherein the capsule contains 25 mg of 1,3-dihydro-4-methyl-5-[4-(methylthio)-benzoyl]-2H-imidazol-2-one.

13. A composition of any one of Claims 1 to 8 wherein the capsule contains 37.5 mg of 1,3-dihydro-4-methyl-5-[4-(methylthio)-benzoyl]-2H-imidazol-2-one.

14. A composition of any one of Claims 1 to 8 wherein the capsule contains 50 mg of 1,3-dihydro-4-methyl-5-[4-(methylthio)-benzoyl]-2H-imidazol-2-one.

15. A composition of any one of Claims 1 to 8 wherein the capsule contains 75 mg of 1,3-dihydro-4-methyl-5-[4-(methylthio)-benzoyl]-2H-imidazol-2-one.

16. A composition of any one of Claims 1 to 8 wherein the capsule contains 100 mg of 1,3-dihydro-4-methyl-5-[4-(methylthio)-benzoyl]-2H-imidazol-2-one.

17. A composition of any one of Claims 1 to 8 wherein the capsule contains 150 mg of 1,3-dihydro-4-methyl-5-[4-(methylthio)-benzoyl]-2H-imidazol-2-one.

18. A process for preparing a liquid pharmaceutical composition according to claims 1-17 consisting of admixing

a) an effective amount of a 4-aroylimidazol-2-one, or a pharmaceutically acceptable salt thereof, and

b) one or more pharmaceutically acceptable nonionic surfactants is an amount from 30% to 99% by weight of the composition.

**Claims for the following Contracting States: ES GR**

1. A.Process for preparing a liquid pharmaceutical composition for use in oral administration dosage forms consisting of admixing a) an effective amount of a 4-aroylimidazol-2-one, or a pharmaceutically acceptable salt thereof, and b) one or more pharmaceutically acceptable nonionic surfactants in an amount from 30% to 99% by weight of the composition.

2. A process of Claim 1 wherein the 4-aroylimidazol-2-one is 1,3-dihydro-4-methyl-5-[4-(methylthio)-benzoyl]-2H-imidazol-2-one.

3. A process of Claim 2 wherein a nonionic surfactant is polyoxyethylene (20) sorbitan monooleate.

4. A process of Claim 3 wherein the amount of the nonionic surfactant is from 55% to 75% by weight of the composition.

5. A process of Claim 4 wherein the amount of the nonionic surfactant is from 60% to 70% by weight of the composition.

6. A process of Claim 5 wherein the amount of the nonionic surfactant is 66% by weight of the composition.

**Patentansprüche**

**Patentansprüche für folgende Vertragsstaaten : AT, BE, CH, DE, FR, GB, IT, LI, LU, NL, SE, S et A.**

1. Flüssige pharmazeutische Zusammensetzung zur Verwendung in Dosisformen für die orale Verabreichung, bestehend aus a) einer wirksamen Menge eines 4-Aroylimidazol-2-ons oder eines pharmazeutisch verträglichen Salzes davon und b) einem oder mehreren pharmazeutisch verträglichen nichtionischen Tensiden in einer Menge von 10 bis 99 Gew.% der Zusammensetzung.

2. Zusammensetzung nach Anspruch 1, worin das 4-Aroyl-imidazol-2-on das 1,3-Dihydro-4-methyl-5-[4-(methylthio)-benzoyl]-2H-imidazol-2-on ist.

3. Zusammensetzung nach Anspruch 1 oder 2, worin ein nichtionisches Tensid ein Polyoxyäthylen-sorbitan-fettsäure-ester ist.

4. Zusammensetzung nach Anspruch 1 oder 2, worin ein nichtionisches Tensid das Polyoxyäthylen(20)sorbitan-monooleat ist.

5. Zusammensetzung nach Anspruch 4, worin die Menge des nichtionischen Tensids 55 bis 75 Gew.% der Zusammensetzung beträgt.

6. Zusammensetzung nach Anspruch 4, worin die Menge des nichtionischen Tensids 60 bis 70 Gew.% der Zusammensetzung beträgt.

7. Zusammensetzung nach Anspruch 4, worin die Menge des nichtionischen Tensids 66 Gew.% der Zusammensetzung beträgt.

8. Zusammensetzung nach einem der Ansprüche 5, 6 oder 7 in einer Einzeldosisform einer pharmazeutisch verträglichen Kapsel.

9. Zusammensetzung nach einem der Ansprüche 1 bis 8 in einer Einzeldosisform einer Weichgelatinekapsel.

10. Zusammensetzung nach einem der Ansprüche 1 bis 8 in einer Einzeldosisform einer Hartgelatinekapsel.

11. Zusammensetzung nach einem der Ansprüche 1 bis 8, worin die Kapsel 12,5 mg 1,3-Dihydro-4-methyl-5-[4-(methylthio)-benzoyl]-2H-imidazol-2-on enthält.

12. Zusammensetzung nach einem der Ansprüche 1 bis 8, worin die Kapsel 25 mg 1,3-Dihydro-4-methyl-5-[4-(methylthio)-benzoyl]-2H-imidazol-2-on enthält.

13. Zusammensetzung nach einem der Ansprüche 1 bis 8, worin die Kapsel 17,5 mg 1,3-Dihydro-4-methyl-5-[4-(methylthio)-benzoyl]-2H-imidazol-2-on enthält.

14. Zusammensetzung nach einem der Ansprüche 1 bis 8, worin die Kapsel 50 mg 1,3-Dihydro-4-methyl-5-[4-(methylthio)-benzoyl]-2H-imidazol-2-on enthält.

15. Zusammensetzung nach einem der Ansprüche 1 bis 8, worin die Kapsel 75 mg 1,3-Dihydro-4-methyl-5-[4-(methylthio)-benzoyl]-2H-imidazol-2-on enthält.

16. Zusammensetzung nach einem der Ansprüche 1 bis 8, worin die Kapsel 100 mg 1,3-Dihydro-4-methyl-5-[4-(methylthio)-benzoyl]-2H-imidazol-2-on enthält.

17. Zusammensetzung nach einem der Ansprüche 1 bis 8, worin die Kapsel 150 mg 1,3-Dihydro-4-methyl-5-[4-(methylthio)-benzoyl]-2H-imidazol-2-on enthält.

18. Verfahren zur Herstellung einer flüssigen pharmazeutischen Zusammensetzung gemäß den Ansprüchen 1-17 bestehend aus dem Mischen

a) einer wirksamen Menge eines 4-Aroylimidazol-2-ons oder eines pharmazeutisch verträglichen Salzes davon und

b) eines oder mehrerer pharmazeutisch verträglicher nichtionischer Tenside in einer Menge von 10 bis 99 Gew.% der Zusammensetzung.

## Patentansprüche für folgende Vertragsstaaten : ES, GR

1. Verfahren zur Herstellung einer flüssigen pharmazeutischen Zusammensetzung zur Verwendung in Dosisformen für die orale Verabreichung, bestehend aus dem Mischen a) einer wirksamen Menge eines 4-Aroylimidazol-2-ons oder eines pharmazeutisch verträglichen Salzes davon und b) eines oder mehrerer pharmazeutisch verträglicher nichtionischer Tenside in einer Menge von 30 bis 99 Gew.% der Zusammensetzung.

2. Verfahren nach Anspruch 1, worin das 4-Aroylimidazol-2-on das 1,3-Dihydro-4-methyl-5-[4-(methylthio)-benzoyl]-2H-imidazol-2-on ist.

3. Verfahren nach Anspruch 2, worin ein nichtionisches Tensid das Polyoxyäthylen(20)sorbitan-monooleat ist.

4. Verfahren nach Anspruch 3, worin die Menge des nichtionischen Tensids 55 bis 75 Gew.% der Zusammensetzung beträgt.

5. Verfahren nach Anspruch 4, worin die Menge des nichtionischen Tensids 60 bis 70 Gew.% der Zusammensetzung beträgt.

6. Verfahren nach Anspruch 5, worin die Menge des nichtionischen Tensids 66 Gew.% der Zusammensetzung beträgt.

## Revendications

### Revendications pour les Etats contractants suivants : AT, BE, CH, DE, FR, GB, IT, LI, LU, NL, SE, S et A.

1. Une composition pharmaceutique liquide pour l'utilisation dans des formes de dosage pour l'administration orale consistant en a) une quantité efficace d'une 4-aroylimidazole-2-one, ou d'un de ses sels pharmaceutiquement acceptables et b) un ou plusieurs tensioactifs non ioniques pharmaceutiquement acceptables en quantité de 30 à 99 % en poids de la composition.

2. Une composition selon la revendication 1, dans laquelle la 4-aroylimidazole-2-one est la 1,3-dihydro-4-méthyl-5-[4-(méthylthio)-benzoyl]-2H-imidazole-2-one.

3. Une composition selon la revendication 1 ou 2, dans laquelle un tensioactif non ionique est un ester d'acide gras de polyoxyéthylènesorbitan.

4. Une composition selon la revendication 1 ou 2, dans laquelle un tensioactif non ionique est le monooléate de polyoxyéthylène (20) sorbitan.

5. Une composition selon la revendication 4, dans laquelle la quantité du tensioactif non ionique est de 55 à 75 % en poids de la composition.

6. Une composition selon la revendication 4, dans laquelle la quantité du tensioactif non ionique est de 60 à 70 % en poids de la composition.

7. Une composition selon la revendication 4, dans laquelle la quantité du tensioactif non ionique est de 66 % en poids de la composition.

8. Une composition selon l'une des revendications 5, 6 ou 7, sous forme de dosage unitaire d'une capsule acceptable en pharmacie.

9. Une composition selon l'une quelconque des revendications 1 à 8 dans une forme de dosage unitaire d'une capsule de gélatine molle.

10. Une composition selon l'une quelconque des revendications 1 à 8 dans une forme de dosage unitaire d'une capsule de gélatine dure.

11. Une composition selon l'une quelconque des revendications 1 à 8, dans laquelle la capsule contient 12,5 g de 1,3-dihydro-4-méthyl-5-[4-(méthylthio)-benzoyl]-2H-imidazole-2-one.

12. Une composition selon l'une quelconque des revendications 1 à 8, dans laquelle la capsule contient 25 mg de 1,3-dihydro-4-méthyl-5-[4-(méthylthio)-benzoyl]-2H-imidazole-2-one.

13. Une composition selon l'une quelconque des revendications 1 à 8, dans laquelle la capsule contient 37,5 mg de 1,3-dihydro-4-méthyl-5-[4-(méthylthio)-benzoyl]-2H-imidazole-2-one.

14. Une composition selon l'une quelconque des revendications 1 à 8, dans laquelle la capsule contient 50 mg de 1,3-dihydro-4-méthyl-5-[4-(méthylthio)-benzoyl]-2H-imidazole-2-one.

15. Une composition selon l'une quelconque des revendications 1 à 8, dans laquelle la capsule contient 75 mg de 1,3-dihydro-4-méthyl-5-[4-(méthylthio)-benzoyl]-2H-imidazole-2-one.

16. Une composition selon l'une quelconque des revendications 1 à 8, dans laquelle la capsule contient 100 mg de 1,3-dihydro-4-méthyl-5-[4-(méthylthio)-benzoyl]-2H-imidazole-2-one.

17. Une composition selon l'une quelconque des revendications 1 à 8, dans laquelle la capsule contient 150 mg de 1,3-dihydro-4-méthyl-5-[4-(méthylthio)-benzoyl]-2H-imidazole-2-one.

18. Un procédé pour préparer une composition pharmaceutique liquide selon les revendications 1 à 17 consistant à mélanger

a) une quantité efficace d'une 4-aroylimidazole-2-one ou d'un de ses sels pharmaceutiquement acceptables et

b) un ou plusieurs tensioactifs non ioniques pharmaceutiquement acceptables en quantité de 30 à 99 % en poids de la composition.

### Revendications pour les Etats contractants suivants : ES, GR

1. Un procédé pour préparer une composition pharmaceutique liquide à utiliser dans des formes de dosage pour l'administration orale consistant à mélanger a) une quantité efficace d'une 4-aroylimidazole-2-one ou d'un de ses sels pharmaceutiquement acceptables et b) un ou plusieurs tensioactifs non ioniques pharmaceutiquement acceptables en quantité de 30 à 99 % en poids de la composition.

2. Un procédé selon la revendication 1, dans lequel la 4-aroylimidazole-2-one est la 1,3-dihydro-4-méthyl-5-[4-(méthylthio)-benzoyl]-2H-imidazole-2-one.

3. Un procédé selon la revendication 2, dans lequel un tensioactif non ionique est le monooléate de polyoxyéthylène (20) sorbitan.

4. Un procédé selon la revendication 3, dans lequel la quantité du tensioactif non ionique est de 55 à 75 % en poids de la composition.

5. Un procédé selon la revendication 4, dans lequel la quantité du tensioactif non ionique est de 60 à 70 % en poids de la composition.

6. Un procédé selon la revendication 5, dans lequel la quantité du tensioactif non ionique est de 66 % en poids de la composition.